# EUROPEAN PATENT SPECIFICATION

(11) **EP 3 031 306 B1**
(45) Date of publication and mention of the grant of the patent: **06.09.2017**
(21) Application number: 14777171.1
(22) Date of filing: 29.07.2014
(51) Int. Cl.: H05H 1/30, H05H 1/42, A61B 18/04, A61L 2/14

(54) **DEVICE AND METHOD FOR GENERATING REACTIVE SPECIES BY MEANS OF PLASMA AT ATMOSPHERIC PRESSURE.**
VORRICHTUNG UND VERFAHREN ZUR ERZEUGUNG EINER REAKTIVEN SPEZIES MITTELS EINES PLASMAS BEI ATMOSPHÄRENDRUCK
DISPOSITIF ET PROCÉDÉ DE GÉNÉRATION D'ESPÈCES RÉACTIVES AU MOYEN D'UN PLASMA À LA PRESSION ATMOSPHÉRIQUE

(30) Priority: 06.08.2013 IT BO20130444
(43) Date of publication of application: 15.06.2016
(73) Proprietor: Alma Mater Studiorum -Università di Bologna, 40126 Bologna (IT)
(72) Inventor: COLOMBO, Vittorio, I-40023 Castel Guelfo (Bologna) (IT); GHEDINI, Emanuele, I-40053 Bazzano (Bologna) (IT); SANIBONDI, Paolo, I-42124 Reggio Emilia (IT); GHERARDI, Matteo, I-40128 Bologna (IT); STANCAMPIANO, Augusto, I-40033 Casalecchio di Reno (Bologna) (IT); LAURITA, Romolo, I-85100 Potenza (IT); LIGUORI, Anna, I-87062 Cariati (Cosenza) (IT); BOSELLI, Marco, I-40123 Bologna (IT); FRIDMAN, Alexander, Philadephia, Pennsylvania 19146 (US)
(74) Representative: Conti, Marco
(86) International application number: PCT/IB2014/063500
(87) International publication number: WO 2015/019240

(56) References cited:
- EP-A1- 2 160 081
- WO-A1-2010/107722
- GB-A- 2 454 461
- US-A1- 2004 173 316
- US-A1- 2004 208 805

## Description

### Technical field

This invention relates to a device and a method for generating reactive species by means of plasma at atmospheric pressure.

### Background art

Within the field of devices for generating plasma at atmospheric pressure, the use of induction plasma torches is known, having a plasma generating chamber and a plasma expansion chamber.

For example, patent document US20090186167A1 describes an induction plasma torch in which an inert gas is fed into the plasma chamber and a reactive gas is fed into the expansion chamber.

That torch is designed to treat surfaces of limited size, of around one square millimetre.

Moreover, another disadvantage of such a torch is linked to its limited effectiveness in the treatment of surfaces (or substrates) of biological material (for example, human skin).

In any case, such a torch has a relatively limited capacity for generating reactive species.

Another induction plasma torch is described in patent document US2004/0173316 A1. US20110171188 describes a system for treating sensitive surfaces, for example wounds, using plasma. That system involves the introduction of additives after generation of the plasma. However, even that system does not allow the production of reactive species to be maximized.

Moreover, there are prior art thermal (high-temperatures) plasma generating system.

It should be noticed that such thermal plasma generators are not really suitable for treating heat-sensitive surfaces. In fact, they are used for other purposes, such as cutting, welding or waste treatment.

Therefore, thermal plasma generating systems are not used to produce reactive oxygen and nitrogen species (for therapeutic purposes).

Moreover, the reactive species contained in thermal plasma have a short useful life. Therefore, in order to be able to convey them onto a substrate to be treated, the outfeed of the plasma source must be positioned very close to the surface to be treated, causing the transfer to the substrate of a large amount of heat and simultaneous damage.

### Disclosure of the invention

This invention has for an aim to provide a device and a method for generating plasma at atmospheric pressure which overcome the above-mentioned disadvantages of the prior art.

In particular, the aim of this invention is to provide a device and a method for generating reactive species, particularly of the oxygen and nitrogen types, by means of plasma at atmospheric pressure, able to treat relatively large surfaces (for example, of around several square centimetres).

A further aim of this invention is to provide a device and a method for generating plasma at atmospheric pressure which are particularly effective in the treatment of substrates of heat-sensitive or biological material. These aims are fully achieved by the device and the method according to this invention as characterized in the appended claims.

The device according to the invention is an atmospheric pressure plasma generator device.

The device comprises a plasma generating chamber.

Inside the plasma generating chamber an electric (electromagnetic) field is produced, by means of an electric generator.

It should be noticed that said electric generator may be an integral part of the device (in this case the device comprises the generator). Alternatively, the device (the plasma chamber) is connectable to the generator, which, in that case, is an element outside the device.

Preferably, the plasma generator is of the inductive type. Therefore, the electric generator is designed to operate at high frequencies and generates a high frequency oscillating electromagnetic field.

Moreover, the device comprises a plasma cooling chamber, located downstream of the plasma generating chamber and connected to it. The plasma cooling chamber is configured to cool the plasma and to convey it onto a surface to be treated.

The plasma generating chamber is connected to a source of inert gas for receiving a flux of inert gas.

Moreover, according to the invention, the plasma generating chamber is also connected to a source of reactive gas for receiving a flux of reactive gas.

Therefore, the inert gas and the reactive gas are mixed in the plasma generating chamber.

It should be noticed that, preferably, the electric generator has a power of at least 0.8 kW (preferably at least 1 kW. It should be noticed, for example, that the power of 1 kW at 13 MHz allows 12% air to be mixed with an inert gas such as Ar).

Moreover, preferably, the plasma generating chamber has a cross section at least 10 mm in diameter, preferably 10-20 mm.

Therefore, thanks to said mixing of the inert gas and the reactive gas in the plasma generating chamber, there is a massive (relatively abundant) production of reactive species (already in the plasma generating chamber).

Therefore, the invention allows the massive generation of reactive species (of the oxygen and/or nitrogen type) by means of plasma (thanks to the mixing of the reactive and inert gases in the plasma generating chamber) and allows said reactive species to be made available at the outfeed at a low temperature (for example, around 40°C), thanks to the cooling chamber.

That allows the treatment of heat-sensitive surfaces (in particular biological, for example human skin) having a relatively large area, with massive quantities of reactive species.

It should be noticed that, preferably, the inert gas and the reactive gas are fed into the plasma generating chamber separately.

Therefore, said device constitutes a plasma torch suitable for treating delicate surfaces (thanks to the temperatures involved), but at the same time allows high efficiency in the treatment, thanks to the presence of reactive species.

In light of this, it should be noticed that an outfeed of the device (of the cooling chamber) is positioned close to the zone where the plasma is generated (that is to say, the plasma generating chamber).

A preferred application of this invention is the treatment, on a biological substrate, of an area measuring a few square centimetres.

Preferably, the flux rate of reactive gas entering the plasma chamber is smaller than the flux rate of inert gas entering the plasma chamber.

For example, (where the electric generator frequency is around 13-14MHz and its power in the range 500W-1 kW), the flux rate of reactive gas (for example, air) is preferably around 1 l/min and the flux rate of inert gas (for example, Argon) is 14-20 l/min. Therefore, preferably, the flux rate of the reactive gas, compared with the total flux rate (of reactive and inert gases) is preferably between 5% and 12%.

Advantageously, that allows the generation of a stable plasma and a reduction in energy consumption.

Preferably, the function of the inert gas fed into the plasma generating chamber is to sustain the plasma, whilst the function of the reactive gas fed into the plasma generating chamber is not only to allow the formation of reactive species, but also to cool the walls of the torch, by being suitably conveyed (for that purpose, the plasma generating chamber is equipped with a suitably shaped partition). It should be noticed that the roles of the two gases may also be swapped, directing the inert gas along the walls of the chamber and the reactive gas along the axis of the torch.

Preferably, the plasma cooling chamber is connected to a further source of reactive gas, for receiving a flux of additional reactive gas. Said additional reactive gas is mixed with the plasma after its formation, that is to say, downstream of the plasma generating chamber.

Preferably, the flux rate of said additional reactive gas entering the cooling chamber is (much) greater than the flux rate of inert gas entering the plasma chamber. Preferably, the flux rate of said additional reactive gas entering the cooling chamber is at least twice the flux rate of inert gas entering the plasma chamber.

This allows a discharge of plasma at the torch outfeed to be tolerated by a heat-sensitive substrate, such as a biological substrate.

Preferably, the plasma cooling chamber is also connected to a source of reactive material, for receiving a flux of reactive material (solid, liquid or gaseous) which is mixed with the additional reactive gas.

Therefore, the invention provides a method for generating reactive species (in particular of the oxygen and/or nitrogen type) by means of plasma at atmospheric pressure.

Said method comprising a step of feeding a flux of inert gas into a plasma generating chamber, in which an electric (or rather, electromagnetic) field has been created, and a step of cooling the plasma in a plasma cooling chamber, located downstream of the plasma generating chamber, in order to convey the plasma onto a surface to be treated.

The invention comprises a step of (also) feeding into the plasma generating chamber a flux of reactive gas, in order to mix the reactive gas with the inert gas in the plasma generating chamber and to generate reactive species in the plasma.

Therefore, in use, the invention provides said device or said method for treating a biological substrate (for example, human skin).

### Brief description of the drawings

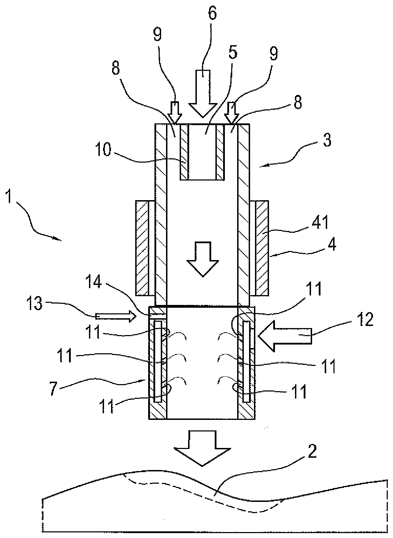

This and other features of the invention will become more apparent from the following detailed description of a preferred embodiment of it illustrated by way of non-limiting example in the accompanying drawing in which the single figure illustrates a plasma generator device according to this invention.

The numeral 1 in the drawing denotes a plasma generator device according to the invention.

### Detailed description of preferred embodiments of the invention

Said device 1 constitutes a plasma torch for the treatment of a substrate 2 (that is to say, a surface 2). The substrate is preferably biological material. The device 1 is an atmospheric pressure plasma generator. Preferably, it is of the induction type.

The device 1 comprises a plasma generating chamber 3. The plasma generating chamber 3 is connected (or connectable) to an electric generator 4 (only partly illustrated, of the known type), designed to create an electric field in the plasma chamber 3.

Preferably, the plasma generator is of the induction type. In light of this, preferably, the electric generator 4 comprises a high-frequency (in particular at a frequency of between 3 and 30 MHz) voltage generator (not illustrated). Moreover, the electric generator 4 comprises a winding 41 connected to said voltage generator. The winding 41 is coupled to the plasma generating chamber 3 for the (high frequency) induction of an electromagnetic field inside the chamber 3.

The plasma generating chamber 3 forms an infeed 5 for receiving a flux of inert gas 6.

Said inert gas comprises a noble or monoatomic gas, for example one or more of the following gases mixed together: argon, helium, nitrogen, neon or krypton.

Moreover, the device 1 comprises a plasma cooling chamber 7, located downstream of the plasma generating chamber 3 and connected there. The plasma cooling chamber 7 is configured to cool the plasma and to convey it onto a surface to be treated.

Preferably, the plasma generating chamber 3 forms a further infeed 8 for receiving a flux 9 of reactive gas. The plasma generating chamber 3 allows mixing of said inert gas 6 and said reactive gas 9 inside it.

The reactive gas 9 is preferably air or oxygen.

Preferably, the flux rate of reactive gas 9 entering the plasma generating chamber 3 is smaller than the flux rate of inert gas 6 entering the plasma chamber. Preferably, the ratio of the flux rate of reactive gas 9 entering the plasma generating chamber 3 and the flux rate of inert gas 6 entering the plasma chamber is around 1/14-1/20.

Preferably, the plasma generating chamber 3 comprises a partition 10 (or a plurality of partitions), close to the infeeds 5 and 8. Said partition 10 is preferably configured to direct the flux of reactive gas 9 along inside walls of the chamber itself in order to cool them. Moreover, preferably, the partition 10 is configured to direct the flux 6 of inert gas along a longitudinal axis of the torch, that is to say, of the plasma generator device 1.

Preferably, the plasma cooling chamber 7 has a plurality of openings 11 (that is to say, transit holes) for receiving a flux of additional reactive gas 12 (additional to the reactive gas 9 fed into the plasma generating chamber 3). Therefore, said additional reactive gas 12 is fed into the device 1 after the reactive gas 9 is fed in.

Preferably, the flux rate of the additional reactive gas 12 entering the cooling chamber 7 is greater (preferably at least twice) the flux rate of inert gas 6 entering the plasma chamber 3.

Preferably, the plasma cooling chamber 7 has a further passage 14 for feeding reactive material 13 into the plasma cooling chamber 7. Said reactive material 13 is mixed with said additional reactive gas 12 and with said mixture of the reactive gas 9 and the inert gas 6 arriving from the plasma generating chamber 3.

Preferably, said openings 11 (or nozzles) are angled in such a way that their stretches communicating with an inner space of the plasma cooling chamber 7 are oriented in the opposite direction to the direction in which the gases 6, 9 and the plasma pass through the device 1 (as they move towards an outfeed of the device 1).

Regarding the discharge of the plasma from the device 1, the plasma cooling chamber comprises, for example, a gas diffuser, or a porous membrane, or at least one annular orifice, or at least one hole (these are of the known type and therefore are not illustrated).

Therefore, at least two flux rates of mixtures of inert and reactive gases are supplied at the plasma torch 1 infeed, for generating reactive species by means of plasma generation (with inductive coupling).

In fact, the presence of a reactive gas in the plasma generating zone allows the formation of reactive species, such as "oxygen radicals" and/or "nitrogen radicals".

Said flux of further reactive gas further increases the production of reactive species and significantly reduces the temperature of the gas flux (to around 40°C).

The reactive species are then conveyed, at the device 1 outfeed, over an area of a few square centimetres of a biological substrate 2 for the plasma treatment.

Said further reactive gas may comprise one of the following gases or a mixture of them: oxygen, nitrogen or air.

Said reactive material fed into the plasma cooling chamber 7 comprises for example collagen (in a solid, liquid or gaseous phase). It is preferably added to the mixture of further reactive gas 12, for depositing the material 13 on the biological substrate 2.

It should be noticed that the mixture of gases 6, 9 in the plasma generating chamber 3 is at a temperature of several thousand degrees Kelvin.

## Claims

1. A reactive species generator device (1) based on plasma at atmospheric pressure, comprising:
- a plasma generating chamber (3), connected to an electric generator (4) designed to create an electric field in the plasma generating chamber (3) and connected also to a source of inert gas (6) for receiving a flux of inert gas (6);
- a plasma cooling chamber (7), located downstream of the plasma generating chamber (3) and configured to cool the plasma and convey it onto a substrate (2) to be treated,
wherein the plasma generating chamber (3) is also connected to a source of reactive gas (9) for receiving a flux of reactive gas (9) in order to mix the inert gas (6) and the reactive gas (9) in the plasma generating chamber (3), thereby generating the reactive species,
**characterized in that** the plasma cooling chamber (7) is connected to a further source of reactive gas in order to receive a flux of additional reactive gas (12).

2. The device according to claim 1, wherein the flux rate of reactive gas (9) entering the plasma generating chamber (3) is smaller than the flux rate of inert gas (6) entering the plasma generating chamber (3).

3. The device according to claim 2, wherein the flux rate of reactive gas (9) entering the plasma generating chamber (3) is approximately 5-12% of the flux rate of inert gas (6) entering the plasma generating chamber (3).

4. The device according to any of the preceding claims, wherein the plasma generating chamber (3) comprises a partition (10) configured to direct the flux of reactive gas (9) along inside walls of the chamber (3) itself in order to cool them, and the flux of inert gas (6) along a longitudinal axis of the device, or vice versa.

5. The device according to any of the preceding claims, wherein the flux rate of the additional reactive gas (12) entering the cooling chamber (7) is at least twice the flux rate of inert gas (6) entering the plasma chamber (3).

6. The device according to any of the preceding claims, wherein the plasma cooling chamber (7) is also connected to a source of reactive material (13) to receive a flux of reactive material (13) for mixing with the additional reactive gas (12).

7. The device according to any of the preceding claims, wherein the plasma cooling chamber (7) comprises a gas diffuser, or a porous membrane, or at least one annular orifice or at least one hole.

8. The device according to any of the preceding claims, wherein the electric generator (4) has a power of at least 0.8 kW and the plasma generating chamber (3) has a cross section at least 10 mm in diameter.

9. The device according to any of the preceding claims, wherein the reactive gas (9) comprises oxygen or air and the reactive species are reactive oxygen species and/or reactive nitrogen species.

10. A method for generating reactive species by means of plasma at atmospheric pressure, comprising the following steps:
- feeding a flux of inert gas (6) into a plasma generating chamber (3) where an electric field has been created;
- cooling the plasma in a plasma cooling chamber (7), located downstream of the plasma generating chamber (3) in order to convey the plasma onto a substrate (2) to be treated,
- feeding into the plasma generating chamber (3) a flux of reactive gas (9) in order to mix the reactive gas (9) with the inert gas (6) in the plasma generating chamber (3) and to generate reactive species in the plasma, **characterized in that** it comprises a step of feeding a flux of additional reactive gas (12) into the cooling chamber (7) after feeding the reactive gas (9) and the inert gas (6).

11. The method according to claim 10, wherein the flux rate of reactive gas (9) entering the plasma generating chamber (3) is approximately 5-12% of the flux rate of inert gas (6) entering the plasma chamber.

12. The method according to claim 10 or 11, wherein the reactive gas (9) is directed along walls of the chamber (3) itself in order to cool them, and the flux of inert gas (6) is directed along a longitudinal axis of the device (1), or vice versa.

13. The method according to any of the claims from 10 to 12, wherein the flux rate of the additional reactive gas (12) entering the cooling chamber (7) is at least twice the flux rate of inert gas (6) entering the plasma chamber (3).

14. The method according to any of the preceding claims, comprising a step of feeding into the plasma cooling chamber (7) a flux of reactive material (13) for mixing with the additional reactive gas (12).

15. Use of a device (1) according to any of the claims from 1 to 9 or of a method according to any of the claims from 10 to 14 to treat a biological substrate (2).

## Patentansprüche

1. Generatorvorrichtung reaktiver Spezies (1) basierend auf Plasma bei Atmosphärendruck, umfassend:
- eine Plasmaerzeugungskammer (3), die mit einem elektrischen Generator (4) verbunden ist, der ausgelegt ist, ein elektrisches Feld in der Plasmaerzeugungskammer (3) zu erzeugen und die auch mit einer Inertgasquelle (6) zur Aufnahme eines Inertgasflusses (6) verbunden ist;
- eine Plasmakühlkammer (7), die stromabwärts der Plasmaerzeugungskammer (3) angeordnet ist und so konfiguriert ist, dass sie das Plasma abkühlt und es auf ein zu behandelndes Substrat (2) transportiert,
wobei auch die Plasmaerzeugungskammer (3) mit einer Reaktivgasquelle (9) zur Aufnahme eines Flusses von Reaktivgas (9) verbunden ist, um das Inertgas (6) und das Reaktivgas (9) in der Plasmaerzeugungskammer (3) zu mischen, wodurch die reaktiven Spezies erzeugt werden, **dadurch gekennzeichnet, dass** die Plasmakühlkammer (7) mit einer weiteren Reaktivgasquelle verbunden ist, um einen Fluss von zusätzlichem Reaktivgas (12) zu erhalten.

2. Vorrichtung nach Anspruch 1, wobei die in die Plasmaerzeugungskammer (3) eintretende Flussrate des Reaktivgases (9) kleiner als die in die Plasmaerzeugungskammer (3) eintretende Flussrate des Inertgases (6) ist.

3. Vorrichtung nach Anspruch 2, wobei die in die Plasmaerzeugungskammer (3) eintretende Flussrate des Reaktivgases (9) etwa 5-12% der in die Plasmaerzeugungskammer (3) eintretende Flussrate des Inertgases (6) beträgt.

4. Vorrichtung nach einem der vorhergehenden Ansprüche, wobei dies Plasmaerzeugungskammer (3) eine Trennwand (10) aufweist, die so konfiguriert ist, um den Fluss des Reaktivgases (9) entlang der Innenwände der Kammer (3) selbst, um sie abzukühlen, und den Fluss des Inertgases (6) entlang einer Längsachse der Vorrichtung leitet oder umgekehrt.

5. Vorrichtung nach einem der vorhergehenden Ansprüche, wobei die Flussrate des in die Kühlkammer (7) eintretenden zusätzlichen Reaktivgases (12) mindestens das doppelte der Flussrate des in die Plasmakammer (3) eintretenden Inertgases (6) ist.

6. Vorrichtung nach einem der vorhergehenden Ansprüche, wobei die Plasmakühlkammer (7) auch mit einer Quelle von reaktivem Material (13) verbunden ist, um einen Fluss von reaktivem Material (13) zum Mischen mit dem zusätzlichen reaktiven Gas (12) zu erhalten.

7. Vorrichtung nach einem der vorhergehenden Ansprüche, wobei die Plasmakühlkammer (7) einen Gasdiffusor oder eine poröse Membran oder mindestens eine ringförmige Öffnung oder mindestens ein Loch aufweist.

8. Vorrichtung nach einem der vorhergehenden Ansprüche, wobei der elektrische Generator (4) eine Leistung von mindestens 0,8 kW aufweist und die Plasmaerzeugungskammer (3) einen Querschnitt von mindestens 10 mm Durchmesser aufweist.

9. Vorrichtung nach einem der vorhergehenden Ansprüche, wobei das Reaktivgas (9) Sauerstoff oder Luft umfasst und die reaktiven Spezies reaktive Sauerstoffspezies und/oder reaktive Stickstoffspezies sind.

10. Verfahren zur Erzeugung reaktiver Spezies mittels Plasma bei Atmosphärendruck, mit folgenden Schritten:
- Zuführen eines Flusses von Inertgas (6) in eine Plasmaerzeugungskammer (3), in der ein elektrisches Feld erzeugt wurde;
- Abkühlen des Plasmas in einer Plasmakühlkammer (7), die stromabwärts der Plasmaerzeugungskammer (3) angeordnet ist, um das Plasma auf ein zu behandelndes Substrat (2) zu transportieren,
- Zuführen in die Plasmaerzeugungskammer (3) eines Flusses vom Reaktivgas (9), um das Reaktivgas (9) mit dem Inertgas (6) in der Plasmaerzeugungskammer (3) zu mischen und reaktive Spezies im Plasma zu erzeugen, **dadurch gekennzeichnet, dass** es einen Schritt des Zuführens eines Flusses von zusätzlichem Reaktivgas (12) in die Kühlkammer (7) nach dem Zuführen des Reaktivgases (9) und des Inertgases (6) umfasst.

11. Verfahren nach Anspruch 10, wobei die in die Plasmaerzeugungskammer (3) eintretende Flussrate des Reaktivgases (9) etwa 5-12% der in die Plasmakammer eintretende Flussrate des Inertgases (6) beträgt.

12. Verfahren nach Anspruch 10 oder 11, wobei das Reaktivgas (9) entlang der Wände der Kammer (3) selbst geleitet wird, um es abzukühlen, und der Fluss des Inertgases (6) wird entlang einer Längsachse der Vorrichtung (1) geleitet oder umgekehrt.

13. Verfahren nach einem der vorhergehenden Ansprüche 10 bis 12, wobei die Flussrate des in die Kühlkammer (7) eintretenden zusätzlichen Reaktivgases (12) mindestens das doppelte der Flussrate des in die Plasmakammer (3) eintretenden Inertgases (6) ist.

14. Verfahren nach einem der vorhergehenden Ansprüche, umfassend einen Schritt des Zuführens in die Plasmakühlkammer (7) eines Flusses von reaktivem Material (13) zum Mischen mit dem zusätzlichen Reaktivgas (12).

15. Verwendung einer Vorrichtung (1) nach einem der Ansprüche 1 bis 9 oder eines Verfahrens nach einem der Ansprüche 10 bis 14 zur Behandlung eines biologischen Substrats (2).

## Revendications

1. Dispositif (1) générateur d'espèces réactives basé sur un plasma à la pression atmosphérique, comprenant :
- une chambre de génération de plasma (3) reliée à un générateur électrique (4) conçu pour créer un champ électrique dans la chambre de génération de plasma (3) et reliée aussi à une source de gaz inerte (6) pour recevoir un flux de gaz inerte (6) ;
- une chambre de refroidissement de plasma (7) située en aval de la chambre de génération de plasma (3) et configurée pour refroidir le plasma et l'acheminer sur un substrat (2) à traiter,
dans lequel la chambre de génération de plasma (3) est aussi reliée à une source de gaz réactif (9) pour recevoir un flux de gaz réactif (9) afin de mélanger le gaz inerte (6) et le gaz réactif (9) dans la chambre de génération de plasma (3), générant ainsi les espèces réactives,
**caractérisé en ce que** la chambre de refroidissement de plasma (7) est reliée à une source de gaz réactif supplémentaire afin de recevoir un flux de gaz réactif (12) supplémentaire.

2. Dispositif selon la revendication 1, dans lequel la vitesse du flux de gaz réactif (9) entrant dans la chambre de génération de plasma (3) est inférieure à la vitesse du flux de gaz inerte (6) entrant dans la chambre de génération de plasma (3).

3. Dispositif selon la revendication 2, dans lequel la vitesse du flux de gaz réactif (9) entrant dans la chambre de génération de plasma (3) correspond approximativement à 5 à 12 % de la vitesse du flux de gaz inerte (6) entrant dans la chambre de génération de plasma (3).

4. Dispositif selon l'une quelconque des revendications précédentes, dans lequel la chambre de génération de plasma (3) comprend une séparation (10) configurée pour diriger le flux de gaz réactif (9) le long des parois intérieures de la chambre (3) elle-même afin de les refroidir, et le flux de gaz inerte (6) le long d'un axe longitudinal du dispositif ou vice versa.

5. Dispositif selon l'une quelconque des revendications précédentes, dans lequel la vitesse du flux de gaz réactif (12) supplémentaire entrant dans la chambre de refroidissement (7) correspond à au moins deux fois la vitesse du flux de gaz inerte (6) entrant dans la chambre de plasma (3).

6. Dispositif selon l'une quelconque des revendications précédentes, dans lequel la chambre de refroidissement de plasma (7) est aussi reliée à une source de matière réactive (13) pour recevoir un flux de matière réactive (13) à mélanger au gaz réactif (12) supplémentaire.

7. Dispositif selon l'une quelconque des revendications précédentes, dans lequel la chambre de refroidissement de plasma (7) comprend un diffuseur de gaz ou une membrane poreuse ou au moins un orifice annulaire ou au moins un trou.

8. Dispositif selon l'une quelconque des revendications précédentes, dans lequel le générateur électrique (4) possède une puissance d'au moins 0,8 kW et la chambre de génération de plasma (3) comporte une section transversale d'au moins 10 mm de diamètre.

9. Dispositif selon l'une quelconque des revendications précédentes, dans lequel le gaz réactif (9) comprend de l'oxygène ou de l'air et les espèces réactives sont des espèces réactives de l'oxygène et/ou des espèces réactives de l'azote.

10. Procédé de génération d'espèces réactives au moyen d'un plasma à la pression atmosphérique, comprenant les étapes suivantes :
- alimenter un flux de gaz inerte (6) dans une chambre de génération de plasma (3) où un champ électrique a été créé ;
- refroidir le plasma dans une chambre de refroidissement de plasma (7) située en aval de la chambre de génération de plasma (3) afin d'acheminer le plasma sur un substrat (2) à traiter,
- alimenter dans la chambre de génération de plasma (3) un flux de gaz réactif (9) afin de mélanger le gaz réactif (9) au gaz inerte (6) dans la chambre de génération de plasma (3) et pour générer des espèces réactives dans le plasma, **caractérisé en ce qu'**il comprend une étape consistant à alimenter un flux de gaz réactif (12) supplémentaire dans la chambre de refroidissement (7) après avoir alimenté le gaz réactif (9) et le gaz inerte (6).

11. Procédé selon la revendication 10, dans lequel la vitesse du flux de gaz réactif (9) entrant dans la chambre de génération de plasma (3) correspond approximativement à 5 à 12 % de la vitesse du flux de gaz inerte (6) entrant dans la chambre de plasma.

12. Procédé selon la revendication 10 ou 11, dans lequel le gaz réactif (9) est dirigé le long des parois de la chambre (3) elle-même afin de les refroidir, et le flux de gaz inerte (6) est dirigé le long d'un axe longitudinal du dispositif (1) ou vice versa.

13. Procédé selon l'une quelconque des revendications de 10 à 12, dans lequel la vitesse du flux du gaz réactif (12) supplémentaire entrant dans la chambre de refroidissement (7) correspond à au moins deux fois la vitesse du flux de gaz inerte (6) entrant dans la chambre de plasma (3).

14. Procédé selon l'une quelconque des revendications précédentes, comprenant une étape consistant à alimenter dans la chambre de refroidissement de plasma (7) un flux de matière réactive (13) à mélanger au gaz réactif (12) supplémentaire.

15. Utilisation d'un dispositif (1) selon l'une quelconque des revendications de 1 à 9 ou selon un procédé selon l'une quelconque des revendications de 10 à 14 pour traiter un substrat biologique (2).
